# EUROPEAN PATENT APPLICATION

(11) **EP 4 043 488 A1**
(43) Date of publication of application: **17.08.2022**
(21) Application number: 21156765.6
(22) Date of filing: 12.02.2021
(51) Int. Cl.: C07K 14/74, C07K 14/705, C07K 16/28

(54) **PARTICLES COMPRISING T-CELL RECEPTOR BINDING MOLECULES AND SLAMF7 MOLECULES**

(71) Applicant: Otto-von-Guericke-Universität Magdeburg, 39106 Magdeburg (DE)
(72) Inventor: Brunner-Weinzierl, Monika, 39104 Magdeburg (DE); Lingel, Holger, 04808 Lossatal (DE)

(57) **Abstract**

The present invention relates to a particle comprising one or more molecules capable of binding a T-cell receptor and one or more molecules comprising an extracellular domain of SLAMF7. Further, the present invention relates to the particle or a composition comprising a plurality of particles for use as medicament, in particular for use in the treatment of cancer, an infection, or an inflammation. The present invention also relates to a combination comprising the particle or the composition and a drug different from the particle or composition for use as medicament, in particular for use in the treatment of cancer, an infection, or an inflammation. Additionally, the invention relates to a kit comprising the particle or composition.

## Description

The present invention relates to a particle comprising one or more molecules capable of binding a T-cell receptor and one or more molecules comprising an extracellular domain of SLAMF7. Further, the present invention relates to the particle or a composition comprising a plurality of particles for use as medicament, in particular for use in the treatment of cancer, an infection, or an inflammation. The present invention also relates to a combination comprising the particle or the composition and a drug different from the particle or composition for use as medicament, in particular for use in the treatment of cancer, an infection, or an inflammation. Additionally, the invention relates to a kit comprising the particle or composition.

### BACKGROUND OF THE INVENTION

In the coming decades, the rising average age alone will lead to a drastic increase in chronic diseases such as cancer and inflammation. In a chronic disease processes, the immune system falls back into a reversible state of exhaustion as a natural defence mechanism.

The conventional procedure for modern immunotherapy is to utilize the body's own immune system against cancer cells. Current methods aim at reactivating the adaptive immune system by means of antibodies against surface molecules that slow down immune processes. The effect of these antibodies can be blocking or depleting for different cell types and can therefore cause considerable side effects without therapeutic success.

Previously approved drugs consist of monoclonal antibodies against surface receptors that systemically regulate so-called immune control points. These antibodies act on any cell that has the appropriate receptor, resulting in respective side effects without therapeutic benefit and can lead, for example, to a systemically hyperactive immune system.

Another strategy is based on bispecific antibodies that can create a spatial proximity between cancer and immune cells. Additionally, the methodology of adoptive T cell transfer is under development. T cells are taken, genetically modified *ex-vivo* so that they form a single model target structure and then reapplied to the patient.

Although many different therapeutic approaches exist, most tumour diseases treated continue to have an infaust course or dramatically reduce the quality of life due to the un-specificity of the medication and thus therapeutic damage to healthy tissue. Therefore, there is a great need for novel therapeutic approaches in immune therapy with less side effects to occur.

In order to develop a more effective, gentle and targeted method, the inventors deactivated the brakes of cancer cell-destroying cytotoxic CD8+ T cells in an experimental ex-*vivo* system and identified potential properties that can be used to specifically increase the immune defence of these cells.

CD8+ T cells are the natural, targeted immune defence against tumours and their metastases. They are located at the end of the activation chain of the immune system. The initial process of CD8+ T cell activation is highly coordinated and is dependent on three main steps:
(1) recognition of cognate MHC-antigen complexes by a T cell receptor (TCR), (2) regulation by costimulatory and -inhibitory receptors and ligands, and (3) binding of cytokines. Furthermore, these signals are integrated during a distinct pattern of dynamic cell interactions involving initial brief contacts to antigen presenting cells (APCs) before forming tight synapses and cell clusters that allow localized cytokine and T cell-T cell coregulation (Krummel et al., 2018).

In CD8+ T cells the present inventors switched off in an novel and innovative experimental setup the immune brakes of the T cells and identified the properties that enable the unleash of the T cells' defence strength against unwanted target cells. Based on these findings, the inventors developed a novel biologic T-cell enhancing method using the innovative combination of the natural ligands SLAMF7 and HLA-A-C.

The human leukocyte antigen (HLA) system or complex is a group of related proteins that are encoded by the major histocompatibility complex (MHC) gene complex in humans. These cell-surface proteins are responsible for the regulation of the immune system. HLA genes are highly polymorphic, which means that they have many different alleles, allowing them to fine-tune the adaptive immune system. The proteins encoded by certain genes are also known as antigens, as a result of their historic discovery as factors in organ transplants. Different classes have different functions: HLAs corresponding to MHC class I (A, B, and C), which all are the HLA Class 1 group, present peptides from inside the cell.

HLA-A-C molecules can be loaded with tumour or virus specific peptides (antigens) and serve to activate the specific cytotoxic CD8+ T cells.

SLAMF7 is a cell surface molecule expressed on hematopoietic cells and involved in various immune-regulatory mechanisms (Boles and Mathew, 2001). It belongs to the SLAM family of surface receptors which are mainly composed of self-ligands. Thus, SLAMF7 can engage itself in trans either between different adjacent cell types (e.g. T-cell:APC) or via homotypic cell interactions. This enables bidirectional signalling and cell regulation. SLAM family receptors can transmit signals through binding of molecules at their intracellular tyrosine motifs. These molecules mainly belong to the signalling lymphocyte activation molecule-associated protein (SAP) adaptors. SLAMF7 is expressed on activated T cells, however it is absent on naive ones (Cruz-Munoz et al., 2009).

The inventors of the present invention discovered the hitherto unknown role of SLAMF7 in the context of initial CD8+ T-cell activation and differentiation. They found that SLAMF7 surface expression was dependent on the activation state and cytotoxicity of CD8+ T cells. Furthermore, they detected a co-regulation of SLAMF7 with CD44 and identified SLAMF7 to be involved in T-cell contact formation, while they found initial T-cell activation and signalling events remained unaffected. Further, they discovered that SLAMF7 engagement caused an accelerated transition from brief to stable T-cell interactions, resulting in a profoundly promoted proliferation.

For the present invention, the inventors innovatively combined the two molecules, SLAMF7 and HLA-A-C by means of specific attachment on e.g. latex particles. The new strategy enables an individually tailored therapy which, *in-vitro,* activates smallest amounts of T-cells and will also trigger a specific CD8+ T-cell response in the body. Due to the precisely targeted effect plus memory formation of this precisely tailored immune response, this new therapeutic method is also directed against future metastases and can be used as vaccine. A critical advantage is the immunological memory of the defence, which dramatically extends the lifespan as well as the health span of the affected persons.

A further application aspect of the particles of the present invention, besides the therapeutic *in-vivo* application, is the use for *ex-vivo* activation and enrichment of CD8+ T cells to perform an adoptive T cell transfer. The particles of the present invention lead to an antigen-specific crosslinking of the T cell receptor and SLAMF7, which lowers the activation threshold and increases the cell division of cytotoxic CD8+ T cells. This effect of the particles enables the recruitment and proliferation of CD8+ T cells that are directed against less potent antigens and thus exploit the full range of the immune response.

When used *in vivo,* the particles of the invention can be switched off at any time by proteolytic cleavage. The particles can be used as a stand-alone therapy or in combination with other drugs to precisely potentiate the natural defence against cancer and provide lifelong protection against metastases.

A further application of the particles of the present invention is their use chronic inflammation and chronic viral infections such as for the healing of SARS-CoV-2 infected persons. The particles can also be used in prophylactic treatment of diseases, i.e. applied in form of a vaccine. Additionally, the particles of the invention can be used as tool in research to visualize even very minor and inconspicuous T-cell responses, that were otherwise not measurable.

### SUMMARY OF THE INVENTION

In a first aspect, the present invention relates to a particle comprising one or more molecules capable of binding a T-cell receptor and one or more molecules comprising an extracellular domain of SLAMF7.

In a second aspect, the present invention relates a composition comprising a plurality of particles of the first aspect.

In a third aspect, the present invention relates to the particle of the first aspect or the composition of the second aspect for use as medicament.

In a fourth aspect, the present invention relates to a combination comprising the particle of the first aspect or the composition of the second aspect and a drug different from the particle or composition for use as medicament.

In a fifth aspect, the present invention relates to the particle of the first aspect or the composition of the second aspect for use in the treatment of cancer, an infection, or an inflammation.

In a sixth aspect, the present invention relates to a combination comprising the particle of the first aspect or the composition of the second aspect and a drug different from the particle or composition for use in the treatment of cancer, an infection, or an inflammation.

In a seventh aspect, the present invention relates to a kit comprising the particle of the first aspect or the composition of the second aspect.

In an eighth aspect, the present invention relates to a vaccine comprising the particle of the first aspect or the composition of the second aspect.

In a ninth aspect, the invention relates to the use of the particle of the first aspect or the composition of the second aspect for *ex-vivo* expansion and/or activation of MHC recognising (and SLAM7 expressing) cells.

In a tenth aspect, the invention relates to a method of expanding the population of MHC recognising (and SLAM7 expressing) cells comprising the steps of contacting a particle of the first aspect of the invention and a sample suspected of comprising MHC recognising (and SLAM7 expressing) cells, and isolating the MHC recognising (and SLAM7 expressing) cells bound to the particle.

In a eleventh aspect, the invention relates to a method for detecting or monitoring the presence and/or activation of MHC recognising (and SLAMF7 expressing) cells comprising the steps of contacting a particle of the first aspect of the invention and a sample (suspected of) comprising MHC recognising (and SLAMF7 expressing) cells, and determining any binding of the particle to the MHC recognising (and SLAMF7 expressing) cells, whereby the binding indicates the presence/activation of MHC recognising (and SLAMF7 expressing) cells.

In an twelfth aspect, the invention is related to a method for establishing a prognosis of a disease, determining the status of a disease, or diagnosing a disease involving MHC recognising (and SLAMF7 expressing) cells comprising the steps of contacting a particle of the first aspect of the invention and a sample (suspected of) comprising MHC recognising (and SLAMF7 expressing) cells, and determining any binding of the particle to the MHC recognising (and SLAMF7 expressing) cells, thereby establishing a prognosis of a disease, determining the status of a disease, or diagnosing a disease involving MHC recognising (and SLAMF7 expressing) cells.

In a thirteenth aspect, the invention relates to a method for determining the effectiveness of a medicament against a disease involving MHC recognising (and SLAMF7 expressing) cells comprising the steps of contacting a particle of the first aspect of the invention and a sample (suspected of) comprising MHC recognising (and SLAMF7 expressing), determining any binding of the particle to the MHC recognising (and SLAMF7 expressing) cells, thereby determining the effectiveness of the medicament.

This summary of the invention does not necessarily describe all features of the present invention. Other embodiments will become apparent from a review of the ensuing detailed description.

### DETAILED DESCRIPTION OF THE INVENTION

### Definitions

Before the present invention is described in detail below, it is to be understood that this invention is not limited to the particular methodology, protocols and reagents described herein as these may vary. It is also to be understood that the terminology used herein is for the purpose of describing particular embodiments only, and is not intended to limit the scope of the present invention which will be limited only by the appended claims. Unless defined otherwise, all technical and scientific terms used herein have the same meanings as commonly understood by one of ordinary skill in the art.

Preferably, the terms used herein are defined as described in "A multilingual glossary of biotechnological terms: (IUPAC Recommendations)", Leuenberger, H.G.W, Nagel, B. and Kölbl, H. eds. (1995), Helvetica Chimica Acta, CH-4010 Basel, Switzerland).

The term "comprise" or variations such as "comprises" or "comprising" according to the present invention means the inclusion of a stated integer or group of integers but not the exclusion of any other integer or group of integers. The term "consisting essentially of' according to the present invention means the inclusion of a stated integer or group of integers, while excluding modifications or other integers which would materially affect or alter the stated integer. The term "consisting of' or variations such as "consists of' according to the present invention means the inclusion of a stated integer or group of integers and the exclusion of any other integer or group of integers.

The terms "a" and "an" and "the" and similar reference used in the context of describing the invention (especially in the context of the claims) are to be construed to cover both the singular and the plural, unless otherwise indicated herein or clearly contradicted by context.

The term "one or more", as used everywhere herein, is intended to include one and a plurality. This applies, for example, to the molecules comprising an extracellular domain of SLAMF7 and to the molecules capable of binding a T-cell receptor. The particle may therefore have attached thereto one molecule capable of binding a T-cell receptor or a plurality of molecules capable of binding a T-cell receptor and/or one molecule comprising an extracellular domain of SLAMF7 or a plurality of molecules comprising an extracellular domain of SLAM7. For example, the particle may have attached thereto at least 2, 5, 10, 20, 30, 40, 50, 60, 70, 80, 90, or 100 molecules capable of binding a T-cell receptor and/or at least 2, 5, 10, 20, 30, 40, 50, 60, 70, 80, 90, or 100 molecules comprising an extracellular domain of SLAM7.

The terms "polypeptide" and "protein" are used interchangeably in the context of the present invention. They refer to a long peptide-linked chain of amino acids, e.g. one that is typically 40 amino acids long or longer than 40 amino acids.

The term "peptide", as used herein, refers to a short peptide-linked chain of amino acids, e.g. one that is typically less than about 40 amino acids long and more typically less than about 30 amino acids long.

The terms "fusion protein" or "hybrid protein" (literally, made of parts from different sources) are used interchangeably in the context of the present invention. They refer to a protein which is created through the joining of two or more genes that originally coded for separate proteins/peptides. Translation of this fusion gene results in a single chain polypeptide with functional properties derived from each of the original proteins/peptides.

The terms "recombinant fusion protein" or "recombinant hybrid protein" are used interchangeably in the context of the present invention. They refer to a single chain polypeptide which is created artificially by recombinant DNA technology.

The term "antigen", as used herein, relates to a molecule comprising an epitope against which an immune response is to be generated. The term "antigen" includes proteins or peptides. The term "antigen" also includes molecules, which become antigenic only through transformation (e.g. intermediately in the molecule or by completion with body protein). An antigen is preferably presentable by cells of the immune system such as antigen presenting cells (APCs) like dendritic cells (DCs) or macrophages. In addition, an antigen or a processing product thereof is preferably recognizable by a T or B cell receptor, or by an immunoglobulin molecule such as an antibody. The antigen may be a disease-associated antigen, such as a tumor antigen, a viral antigen, or a bacterial antigen.

In the context of the present invention, the term "disease-associated antigen" is used in its broadest sense to refer to any antigen associated with a disease. A disease-associated antigen is a molecule which contains epitopes that will stimulate a host's immune system to make a cellular antigen-specific immune response and/or a humoral antibody response against the disease. The disease-associated antigen may therefore, be used for therapeutic purposes. Disease-associated antigens are preferably associated with infection by microbes, typically microbial antigens, or associated with cancer, typically tumors.

The term "disease", as used herein, refers to an abnormal condition that affects the body of an individual. A disease is often construed as a medical condition associated with specific symptoms and signs. A disease may be caused by factors originally from an external source, such as infectious disease, or it may be caused by internal dysfunctions, such as autoimmune diseases. In humans, "disease" is often used more broadly to refer to any condition that causes pain, dysfunction, distress, social problems, or death to the individual afflicted, or similar problems for those in contact with the individual. In this broader sense, it sometimes includes injuries, disabilities, disorders, syndromes, infections, isolated symptoms, deviant behaviors, and atypical variations of structure and function, while in other contexts and for other purposes these may be considered distinguishable categories. Diseases usually affect individuals not only physically, but also emotionally, as contracting and living with many diseases can alter one's perspective on life, and one's personality.

As mentioned above, the disease may be an infectious disease or an inflammatory disease. The disease may also be a cancer disease or simply cancer.

The term "infectious disease", as used herein, refers to any disease which can be transmitted from individual to individual or from organism to organism, and is caused by a microbial agent (e.g. common cold). Infectious diseases are known in the art and include, for example, a viral disease, a bacterial disease, or a parasitic disease. Said diseases are caused by a virus, a bacterium, and a parasite, respectively. In this regard, the infectious disease can be, for example, hepatitis, sexually transmitted diseases (e.g. chlamydia or gonorrhea), tuberculosis, HIV/acquired immune deficiency syndrome (AIDS), diphtheria, hepatitis B, hepatitis C, cholera, severe acute respiratory syndrome (SARS), Severe acute respiratory syndrome coronavirus 2 (SARS-CoV-2) the bird flu, and influenza.

The terms "cancer disease" or "cancer", as used herein, refer to or describe the physiological condition in an individual that is typically characterized by unregulated cell growth. Examples of cancers include, but are not limited to, carcinoma, lymphoma, blastoma, sarcoma, and leukemia. More particularly, examples of such cancers include bone cancer, blood cancer lung cancer, liver cancer, pancreatic cancer, skin cancer, cancer of the head or neck, cutaneous or intraocular melanoma, uterine cancer, ovarian cancer, rectal cancer, cancer of the anal region, stomach cancer, colon cancer, breast cancer, prostate cancer, uterine cancer, carcinoma of the sexual and reproductive organs, Hodgkin's Disease, cancer of the esophagus, cancer of the small intestine, cancer of the endocrine system, cancer of the thyroid gland, cancer of the parathyroid gland, cancer of the adrenal gland, sarcoma of soft tissue, cancer of the bladder, cancer of the kidney, renal cell carcinoma, carcinoma of the renal pelvis, neoplasms of the central nervous system (CNS), neuroectodermal cancer, spinal axis tumors, glioma, meningioma, and pituitary adenoma. The term "cancer" according to the invention also comprises cancer metastases.

As mentioned above, the antigen may be a tumor antigen, a viral antigen, or a bacterial antigen.

The term "tumor antigen", as used herein, refers to a constituent of cancer cells which may be derived from the cytoplasm, the cell surface, and the cell nucleus. In particular, it refers to those antigens which contain mutations or are produced, preferably in large quantity, intracellularly or as surface antigens on tumor cells. Examples for tumor antigens include HER2, EGFR, VEGF, CAMPATH1-antigen, CD22, CA-125, HLA-DR, Hodgkin-lymphoma or mucin-1, but are not limited thereto.

The term "viral antigen", as used herein, refers to any viral component having antigenic properties, i.e. being able to provoke an immune response in an individual. The viral antigen may be a viral ribonucleoprotein or an envelope protein.

The term "microbial antigen", as used herein, refers to any microbial component having antigenic properties, i.e. being able to provoke an immune response in an individual.

The term "bacterial antigen", as used herein, refers to any bacterial component having antigenic properties, i.e. being able to provoke an immune response in an individual. The bacterial antigen may be derived from the cell wall or cytoplasm membrane of a bacterium.

The term "fungal antigen", as used herein, refers to any fungal component having antigenic properties, i.e. being able to provoke an immune response in an individual.

The term "zooparasitic antigen", as used herein, refers to any component of a parasite of an animal having antigenic properties, i.e. being able to provoke an immune response in an individual. Said parasite may be a flea, louse, or worm.

The term "immune response", as used herein, relates to a reaction of the immune system to immunogenic organisms, such as bacteria, viruses, cells or substances.

The term "immune cells", as used herein, refers to cells of the immune system which are involved in defending the body of an individual. The term "immune cells" encompasses specific types of immune cells and their precursors including leucocytes comprising macrophages, monocytes (precursors of macrophages), granulocytes such as neutrophils, eosinophils and basophils, dendritic cells, mast cells, and lymphocytes such as B cells, T cells and natural killer (NK) cells. Macrophages, monocytes (precursors of macrophages), neutrophils, dendritic cells (DCs), and mast cells are phagocytic cells.

The term "antigen presenting cell (APC)", as used herein, is a cell of a variety of cells capable of displaying, acquiring, and/or presenting at least one antigen or antigenic fragment on (or at) its cell surface. Antigen-presenting cells can be distinguished in professional antigen presenting cells and non-professional antigen presenting cells.

The term "professional antigen presenting cells", as used herein, relates to antigen presenting cells which constitutively express the Major Histocompatibility Complex class II (MHC class II) molecules required for interaction with naive T cells. If a T cell interacts with the MHC class II molecule complex on the membrane of the antigen presenting cell, the antigen presenting cell produces a co-stimulatory molecule inducing activation of the T cell. Professional antigen presenting cells comprise dendritic cells (DCs) and macrophages.

The term "non-professional antigen presenting cells", as used herein, relates to antigen presenting cells which do not constitutively express MHC class II molecules, but upon stimulation by certain cytokines such as interferon-gamma. Exemplary, non-professional antigen presenting cells include fibroblasts, thymic epithelial cells, thyroid epithelial cells, glial cells, pancreatic beta cells or vascular endothelial cells.

The term "human leukocyte antigen (HLA) system or complex ", as used herein, refers to a group of related proteins that are encoded by the major histocompatibility complex (MHC) gene complex in humans. These cell-surface proteins are responsible for the regulation of the immune system. HLA genes are highly polymorphic, which means that they have many different alleles, allowing them to fine-tune the adaptive immune system. The proteins encoded by certain genes are also known as antigens, as a result of their historic discovery as factors in organ transplants. Different classes have different functions: HLAs corresponding to MHC class I (A, B, and C), which all are the HLA Class 1 group, present peptides from inside the cell.

The term "Major histocompatibility Complex (MHC)", as used herein, refers to a large locus on vertebrate DNA containing a set of closely linked polymorphic genes that code for cell surface proteins essential for the adaptive immune system. The term "MHC" encompasses MHC molecules.

By the term "Major histocompatibility Complex (MHC) molecule", as used everywhere herein, is meant such molecule, which is capable of performing at least one of the functions attributed to said molecule.

The terms "MHC molecule", and "MHC" are used interchangeably herein.

The term "MHC molecule" is further intended to include MHC Class I molecules, MHC Class II molecules, as well as MHC-like molecules (both Class I and Class II), including the subgroup of non-classical MHC Class I and Class II molecules. In particular, Class I and class II MHC molecules are proteins encoded by the Major histocompatibility Complex (MHC). They function as peptide receptors that display antigens on the cell surface for the surveillance by T cells. Upon recognition, these antigens can trigger the destruction of the cell, so that they had become a focal point for experimental tumour immune therapies.

The terms "T cells" or "T lymphocytes", as used herein, relate to types of lymphocytes that play a central role in cell-mediated immunity. T cells or T lymphocytes can be distinguished from other lymphocytes, such as B cells and natural killer (NK) cells, by the presence of a T cell receptor (TCR) on the cell surface. They do not have antigen presenting properties (but rather, requiring B cells or NK cells for its antigen-presenting property). They are called T cells because they mature in the thymus. T cells are capable of recognizing an antigen when displayed on the surface of antigen presenting cells or matrix together with one or more MHC molecules or one or more non-classical MHC molecules. The T cells are preferably CD4+ or CD8+ T cells.

The terms "stimulating T cells" or "stimulation of T cells", as used herein, refer to the induction or activation of a T cell response by a primary signal, such as by the interaction with an antigen-MHC class II complex through the T cell antigen receptor. The term also includes the co-stimulation of T cells, such as through cytokines (e.g. CD80 or CD86 proteins). A T cell is activated if it has received a primary signaling event which initiates an immune response by the T cell.

The term "priming T cells", as used herein, refers to the induction of a first contact of the T cell with its specific antigen (e.g. by dendritic cells (DCs) presenting the antigen to T cells), which causes the differentiation of the T cell into an effector T cell (e.g. a cytotoxic T cell or a T helper cell).

The terms "expanding T cells" or "expansion of T cells", as used herein, refer to the increase of the number of T cells, preferably T cells specifically recognizing a peptide, e.g. an antigen. It is preferred, that the number of T cells specifically recognizing the peptide or antigen comprised in the particle of the present invention increases. The peptide/antigen is preferably presented in the context of MHC molecules, such as on the surface of antigen presenting cells (APCs) like dendritic cells (DCs) or macrophages.

The term "CD4+ T cells", as used herein, refers to the natural, targeted immune defence against pathogens. CD4 T cells play a global role in directing and regulating other immune cells. They respond to peptides presented on MHC class II molecules, which are found on antigen presenting cells (APCs). As a whole, they play a major role in instigating and shaping adaptive immune responses.

The term "CD8+ T cells", as used herein, refers to the natural, targeted immune defence against viruses, tumours and their metastases. CD8+ T cells are located at the end of the activation chain of the immune system. The initial process of CD8+ T-cell activation is highly coordinated and is dependent on three main steps: (1) recognition of cognate MHC-antigen complexes by a T cell receptor (TCR), (2) regulation by costimulatory and -inhibitory receptors and ligands, and (3) binding of cytokines. Furthermore, these signals are integrated during a distinct pattern of dynamic cell interactions involving initial brief contacts to antigen presenting cells (APCs) before forming tight synapses and cell clusters that allow localized cytokine and T cell-T cell coregulation.

The particle of the present invention comprises one or more molecules capable of binding a T-cell receptor. The term "T-cell receptor (TCR)", as used herein refers to a protein complex found on the surface of T cells, or T lymphocytes, that is responsible for recognizing fragments of antigen as peptides bound to Major histocompatibility Complex (MHC) molecules. The T-cell receptor is preferably consisting of an alpha and a beta chain and accompanied by a CD4+ or CD8+ T-cell co-receptor. The one or more molecules capable of binding a T-cell receptor are preferably selected from the group consisting of MHC I, MHC II, and anti-CD3 molecules.

The term "SLAMF7", as used herein, refers to a molecule which is alternatively named CD319, CS1, CRACC, Novel Ly9, Protein 19A and which is expressed on hematopoietic cells and involved in various immune-regulatory mechanisms. It belongs to the SLAM family of surface receptors which are mainly composed of self-ligands. Therefore, SLAMF7 can engage itself in trans either between different adjacent cell types (e.g. T-cell: APC) or via homotypic cell interactions. This enables bidirectional signalling and cell regulation. SLAM family receptors can transmit signals through binding of molecules at their intracellular tyrosine motifs. These molecules contain SH2 domains and mainly belong to the signalling lymphocyte activation molecule-associated protein (SAP) adaptors. SLAMF7 is expressed on activated T cells, however it is absent on naive ones. SLAMF7 consists of two extracellular immunoglobulin domains (V and C2 domain), a transmembrane domain, and intracellular tyrosine motifs containing a signalling domain. The gene encoding human SLAMF7 is located within the SLAM locus on human chromosome 1q23.

The particle of the present invention comprises one or more molecules comprising at least the extracellular domain of SLAM7. The term "extracellular domain", as used herein, refers to the part of SLAM7 capable of protruding from the outer membrane of the cell organelles and cells, especially permitting the binding to SLAMF7 extracellular domains and thereby enabling SLAMF7 signalling. The one or more molecules comprising an extracellular domain of SLAMF7 are preferably selected from the group consisting of SLAMF7 molecules, anti-SLAMF7 antibodies, and recombinant SLAMF7 molecules.

The term "immunotherapy", as used herein, relates to the treatment of a disease or condition by inducing, enhancing, or suppressing an immune response. Immunotherapies designed to elicit or amplify an immune response are classified as activation immunotherapies, while immunotherapies that reduce or suppress an immune response are classified as suppression immunotherapies. The term "immunotherapy" includes antigen immunization or antigen vaccination as well as tumor immunization or tumor vaccination. The term "immunotherapy" also relates to the manipulation of immune responses such that inappropriate immune responses are modulated into more appropriate ones in the context of autoimmune diseases such as rheumatic arthritis, allergies, diabetes, or multiple sclerosis.

The terms "immunization" or "vaccination", as used herein, describe the process of administering particles according to the invention, alternatively or additionally, an antigen to an individual with the purpose of inducing an immune response, for example, for therapeutic or prophylactic reasons.

The term "therapeutic treatment", as used herein, relates to any treatment which improves the health status and/or prolongs (increases) the lifespan of an individual. Said treatment may eliminate the disease in an individual, arrest, inhibit, or slow the development of a disease in an individual, decrease the frequency or severity of symptoms in an individual, and/or decrease the recurrence in an individual who currently has or who previously has had a disease.

The terms "prophylactic treatment" or "preventive treatment", as used herein, relate to any treatment that is intended to prevent a disease from occurring in an individual. The terms "prophylactic treatment" or "preventive treatment" are used herein interchangeably.

The terms "protect", "prevent", "prophylactic", "preventive", or "protective", as used herein, relate to the prevention and/or treatment of the occurrence and/or the propagation of a disease, e.g. tumor, in an individual. For example, a prophylactic administration of an immunotherapy, e.g. by administering the particle or the pharmaceutical composition of the present invention, can protect the receiving individual from the development of a tumor. For example, a therapeutic administration of an immunotherapy, e.g. by administering the particle or the pharmaceutical composition of the present invention, can stop the development of a disease, e.g. lead to the inhibition of the progress/growth of a tumor. This comprises the deceleration of the progress/growth of the tumor, in particular a disruption of the progression of the tumor, which preferably leads to elimination of the tumor. A therapeutic administration of an immunotherapy may protect the individual from the dissemination or metastasis of existing tumors.

The terms "individual" and "subject" are used interchangeably in the context of the present invention. The individual or subject may be healthy, afflicted with a disease or disorder (e.g. cancer), or susceptible to a disease or disorder (e.g. cancer). The individual or subject may be an animal, e.g. a human. Preferably, the individual or subject is a human or another mammal (e.g. mouse, rat, rabbit, dog, cat, cattle, swine, sheep, horse or primate). Unless otherwise stated, the terms "individual" and "subject" do not denote a particular age and, thus, encompass adults, elderlies, children, and newborns. The "individual" or "subject" may be a "patient".

The term "patient", as used herein, means an individual or subject which is diseased, i.e. which suffers from a disease or disorder. The patient may be an animal, e.g. a human. Preferably, the animal is a human or another mammal (e.g. mouse, rat, rabbit, dog, cat, cattle, swine, sheep, horse or primate).

The particle of the present invention may be administered in the form of any suitable pharmaceutical composition. Said pharmaceutical composition may further comprise adjuvants, pharmaceutical acceptable carriers, diluents, and/or excipients. Said pharmaceutical composition is useful for treating, preventing, or reducing the severity of a disease or disorder. It may be administered locally or systemically, preferably systemically.

The term "systemic administration", a used herein, refers to the administration of the particle of the present invention such that the particle becomes widely distributed in the body of an individual in significant amounts and develops a biological effect (or more specifically the antigen comprised therein). Typical systemic routes of administration include administration by introducing the particle directly into the vascular system or oral, pulmonary, or intramuscular administration wherein the particle is adsorbed, enters the vascular system, and is carried to one or more desired site(s) of action via the blood.

The systemic administration may be by parenteral administration. The term "parenteral administration", as used herein, refers to the administration of the particle of the present invention such that the particle does not pass the intestine. The term "parenteral administration" includes intravenous administration, subcutaneous administration, intradermal administration, or intraarterial administration, but is not limited thereto.

The pharmaceutical compositions of the present invention may comprise adjuvants. The term "adjuvant", as used herein, relates to a compound, which, when administered in combination with an antigen or antigen peptide to an individual, prolongs, enhances, or accelerates an immune response. It is assumed that adjuvants exert their biological activity by one or more mechanisms, including an increase of the surface of the antigen, a prolongation of the retention of the antigen in the body, a retardation of the antigen release, targeting of the antigen to macrophages, increase of the uptake of the antigen, enhancement of antigen processing, stimulation of cytokine release, stimulation and activation of immune cells such as B cells, macrophages, dendritic cells, T cells and unspecific activation of immune cells. Adjuvants comprise a heterogeneous group of compounds such as oil emulsions (e.g., Freund's adjuvants), mineral compounds (such as alum), bacterial products (such as Bordetella pertussis toxin), or immune-stimulating complexes. Examples for adjuvants include saponins, incomplete Freund's adjuvants, complete Freund's adjuvants, tocopherols, or aluminum, but are not limited thereto. However, adjuvants may also have negative side effects. Aluminum salt, for example, has the potential to cause severe local and systemic side-effects including sterile abscesses, eosinophilia and myofascitis, although fortunately most of the more serious side-effects are relatively rare. In addition, there is also community concern regarding the possible role of aluminium in neurodegenerative diseases such as Alzheimer's disease. Consequently, there is a major unmet need for safer and more effective adjuvants suitable for human use. The best would be, if the use of adjuvants would be superfluous.

The pharmaceutical composition according to the present invention is generally applied in a "pharmaceutically effective amount". The term "pharmaceutically effective amount", as used herein, refers to the amount which achieves a desired reaction or a desired effect alone or together with further doses. In case of the treatment of a particular disease, the desired reaction preferably relates to an inhibition of the course of the disease. This comprises slowing down the progress of the disease and, in particular, interrupting or reversing the progress of the disease. The desired reaction in a treatment of a disease may also be a delay of the onset or a prevention of the onset of the disease. An effective amount of the particles or compositions described herein will depend on the condition to be treated, the severeness of the disease, the individual parameters of the patient/subject, including age, physiological condition, size, and weight, the duration of treatment, the type of an accompanying therapy (if present), the specific route of administration, and similar factors. Accordingly, the doses of the particles or compositions described herein may depend on various of such parameters. In case that a reaction in the patient/subject is insufficient with an initial dose, higher doses (or effectively higher doses achieved by a different, more localized route of administration) may be used.

As mentioned above, the pharmaceutical composition of the present invention may further comprise pharmaceutical acceptable carriers, diluents, and/or excipients.

The term "excipient", as used herein, is intended to indicate all substances in a pharmaceutical composition which are not active ingredients such as binders, lubricants, thickeners, surface active agents, preservatives, emulsifiers, buffers, flavoring agents, or colorants.

The term "diluent", as used herein, relates to a diluting and/or thinning agent. Moreover, the term "diluent" includes a solution, suspension (e.g. liquid or solid suspension) and/or media.

The term "carrier", as used herein, relates to one or more compatible solid or liquid fillers, which are suitable for an administration, e.g. to a human. The term "carrier" relates to a natural or synthetic organic or inorganic component which is combined with an active component in order to facilitate the application of the active component. Preferably, carrier components are sterile liquids such as water or oils, including those which are derived from mineral oil, animals, or plants, such as peanut oil, soy bean oil, sesame oil, sunflower oil, etc. Salt solutions and aqueous dextrose and glycerin solutions may also be used as aqueous carrier compounds.

Pharmaceutically acceptable carriers or diluents for therapeutic use are well known in the pharmaceutical art, and are described, for example, in Remington's Pharmaceutical Sciences, Mack Publishing Co. (A. R Gennaro edit. 1985). Examples of suitable carriers include, for example, magnesium carbonate, magnesium stearate, talc, sugar, lactose, pectin, dextrin, starch, gelatin, tragacanth, methylcellulose, sodium carboxymethylcellulose, a low melting wax, cocoa butter, and the like. Examples of suitable diluents include ethanol, glycerol, and water.

Pharmaceutical carriers, diluents, and/or excipients can be selected with regard to the intended route of administration and standard pharmaceutical practice. The pharmaceutical compositions of the present invention may comprise as, or in addition to, the carrier(s), excipient(s) or diluent(s) any suitable binder(s), lubricant(s), suspending agent(s), coating agent(s), and/or solubilising agent(s). Examples of suitable binders include starch, gelatin, natural sugars such as glucose, anhydrous lactose, free-flow lactose, beta-lactose, corn sweeteners, natural and synthetic gums, such as acacia, tragacanth or sodium alginate, carboxymethyl cellulose, and polyethylene glycol. Examples of suitable lubricants include sodium oleate, sodium stearate, magnesium stearate, sodium benzoate, sodium acetate, sodium chloride, and the like. Preservatives, stabilizers, dyes, and even flavoring agents may be provided in the pharmaceutical composition. Examples of preservatives include sodium benzoate, sorbic acid, and esters of p-hydroxybenzoic acid. Antioxidants and suspending agents may be also used.

The term "average diameter" refers to the mean diameter of the particles and may be calculated by dividing the sum of the diameter of each particle by the total number of particles. Although the term "diameter" is used normally to refer to the maximal length of a line segment passing through the center and connecting two points on the periphery of a spherical object, it is also used herein to refer to the maximal length of a line segment passing through the center and connecting two points on the periphery of particles having a substantial spherical shape or other shapes.

The term "particle", as used herein, may refer to a micro- or nano-sized spherical structure. It is preferred that the particle comprises or consists of a matrix and a surface.

The term "surface", as used herein, defines the outer sphere of the particle, which includes those sphere sections that are directly exposed to the surrounding space, e.g. surrounding medium or body liquid. A surface, thus, delineates the outermost layer of the particle which shares an interface with the surrounding space, e.g. surrounding medium or body liquid.

The term "matrix", as used herein, defines the inner sphere of the particle, which is not the surface, i.e. which, according to the above definition, does not include any interface to the surrounding space, e.g. surrounding medium or body liquid. The matrix is to be understood as a solid sphere having a radius and accordingly a volume usually smaller than that of the particle.

The term "net charge of the surface of the particle" relates to the total sum of charges, such as positive and negative charges, at the surface of the particle. For example, if the particle comprises on its surface a higher number of negative charges than positive charges, the net charge of the surface of the particle is negative. If the particle comprises on its surface a higher number of positive charges than negative charges, the net charge of the surface of the particle is positive. If the particle comprises on its surface an equal number of positive charges and negative charges, the net charge of the surface of the particle is neutral, particularly electroneutral. Thus, the net charge of the surface of the particle according to the present invention can be negative, positive, or neutral.

In the context of the present invention, the term "kit of parts (in short: kit)" is understood to be any combination of at least some of the components identified herein, which are combined, coexisting spatially, to a functional unit, and which can contain further components.

### Embodiments of the invention

The inventors of the present invention surprisingly found new molecular properties that enable the unleash of the T-cells defence strength against unwanted target cells. Based on these unexpected findings, the inventors developed a novel biologic T-cell enhancing method using a combination of the natural ligands SLAMF7 and HLA-A-C. This innovative strategy allows for antigen-specific crosslinking of the T cell receptor and SLAMF7, lowering the activation threshold and increasing the cell division of cytotoxic CD8+ T cells. This in turn, enables the recruitment and proliferation of CD8+ T cells that are directed also against less potent antigens, thus, exploiting the full range of immune response. In this regard, pathogenic antigens that can be recognized by T cells only with a lower affinity will become/obtain the ability to induce stronger adaptive cellular immune responses. With the particles according to the present invention, an individually tailored immune therapy with less side effects is possible, which can be used in the treatment of cancer, infections such as bacterial, viral and fungal infections as well as in the treatment of inflammations.

Thus, in a first aspect, the present invention relates to a particle comprising one or more molecules capable of binding a T-cell receptor and one or more molecules comprising an extracellular domain of SLAMF7.

Preferably, the particle according to the invention is a solid particle. This solid particle may have a solid core and a solid surface.

It is preferred that the particle comprises or consists of a matrix and a surface. Preferably, the matrix is homogenous, more preferably without any clear visible inclusions (e.g. determined via electron microscopy).

It is further preferred that the one or more molecules capable of binding a T-cell receptor and/or the one or more molecules comprising an extracellular domain of SLAMF7 are attached to the surface of the particle.

Due to their inherent properties, the one or more molecules capable of binding a T-cell receptor and/or the one or more molecules comprising an extracellular domain of SLAMF7 are passively attached to the particle, mediated by the hydrophobic character of the particles themselves. Particles according to the invention preferably consist of polymers (e.g. polyesters, poly(methyl methacrylate)), which enables them to firmly adsorb other hydrophobic molecules, such as proteins, nucleic acids, hormones or medical agents via van-der-Waals forces. Particles according to the invention which consist of silica are intrinsically hydrophilic, i.e. bind to other hydrophilic groups via van-der Waals forces.

In addition to the passive attachment, the particles according to the invention may be coated with different reactive surface groups as well as, alternatively or additionally, with linker molecules. These groups and/or molecules then provide for further binding properties, in addition to the intrinsic, passive ones.

Thus, in preferred embodiments according to the invention, the one or more molecules capable of binding a T-cell receptor and/or the one or more molecules comprising an extracellular domain of SLAMF7 are directly or indirectly attached to the surface of the particle.

In a preferred embodiment, the one or more molecules capable of binding a T-cell receptor and/or the one or more molecules comprising an extracellular domain of SLAMF7 had been directly attached to the surface of the particle by a reaction of activated surface moieties with said one or more molecules capable of binding a T-cell receptor and/or the one or more molecules comprising an extracellular domain of SLAMF7.

Details of the preparation of the particles according to the invention can be found elsewhere (Krummel et al., 1995). In brief, a particle solution is incubated with the one or more molecules capable of binding a T-cell receptor and/or the one or more molecules comprising an extracellular domain of SLAMF7. After attachment of these molecules on the particles, these particels are precipitated e.g. by centrifugation and the supernatant containing the unbound molecules is discarded. Before further usage, the particles can be washed or treated again to shut down further unspecific binding.

Said direct attachment to the surface of the particle via activated surface moieties may be a covalent or non-covalent linkage.

For example, negatively charged surface residues, such as sulfate or carboxylate, can bind positively charged molecular groups of proteins by ionic interaction, i.e. resulting in non-covalent binding.

Reversely, proteins can be covalently bound to positively charged carboxylate or aminofunctional particles by using carbodiimide or glutaraldehyde. Sulfonyl surface residues that can bind covalently to amine and sulfhydryl groups of proteins. Another alternative is that aldehyde residues are coupled with 1,3-diaminopropane to establish a covalent aldehyde-amine bond by maleimide-thiol reactions. Feasible binding combinations of activated surface residues to respective groups of target molecules are known to the person skilled in the art.

Thus, in preferred embodiments of the invention in which the one or more molecules capable of binding a T-cell receptor and/or the one or more molecules comprising an extracellular domain of SLAMF7 are directly attached to the particle, the activated surface moieties may be selected from the group consisting of sulfate-, tosyl-, carboxyl-, amino-, hydroxyl-, hydrazide-, chloromethyl-, silanol-, and maleimide-moieties.

Alternatively or additionally, in other embodiments according to the invention, linker molecules can be used for attachment (i.e. indirect attachment), e.g. to ensure the correct orientation of the target molecule to be bound (i.e. the molecules capable of binding a T-cell receptor and/or the molecules comprising an extracellular domain of SLAMF7). For biotinylated target molecules, for example, the particles can be precoated with avidin, streptavidin, neutral avidin or anti-biotin antibodies.

This means that in certain preferred embodiments of the invention, the one or more molecules capable of binding a T-cell receptor and/or the one or more molecules comprising an extracellular domain of SLAMF7 are indirectly attached to the surface of the particle via one or more linker molecules.

In these embodiments, the one or more linker molecules had been directly attached to the surface of the particle by a reaction of activated surface moieties with said one or more linker molecules.

The direct attachment to the surface of the particle via activated surface moieties may be a covalent or non-covalent linkage.

It is preferred that the activated surface moieties that directly attach the linker molecule to the surface of the particle are selected from the group consisting of sulfate- tosyl-, carboxyl-, amino-, hydroxyl-, hydrazide-, chloromethyl-, silanol-, and maleimide-moieties.

Feasible binding combinations of activated surface residues to respective groups of linker molecules are known to the person skilled in the art. For example, negatively charged surface residues, such as sulfate- or carboxyl-, can bind positively charged molecular groups of e.g. antibodies, by ionic-, non-covalent interaction. Furthermore, sulfonyl surface residues that can bind covalently to amine and sulfhydryl groups of proteins, for example.

Preferably, the one or more linker molecules are directly attached to the one or more molecules capable of binding a T-cell receptor and/or the one or more molecules comprising an extracellular domain of SLAMF7.

More preferably, the direct attachment of the linker molecules to the one or more molecules capable of binding a T-cell receptor and/or the one or more molecules comprising an extracellular domain of SLAMF7 is a covalent or non-covalent linkage.

It is preferred that the one or more linker molecules are selected from the group consisting of antibodies, antibody fragments or derivatives thereof, photoreactive conjugates, cleavable linkers, recombinant proteins, and recombinant fusion proteins.

In case that the linker groups are antibodies, it is preferred that the antibodies are monoclonal antibodies, preferably anti-biotin antibodies, or polyclonal antibodies. In case that said linker molecules are recombinant proteins, it is preferred that they are avidin or derivatives thereof, preferably neutravidin, or streptavidin and derivatives thereof. In case the linker molecule is a recombinant fusion protein, it is preferably protein A/G.
In case the linker molecules are photoreactive conjugates, it is preferred that they have a photocleavable 1-(2-nitrophenyl)-ethyl moiety.
In preferred embodiments, the particles of the invention can be switched off at any time by proteolytic cleavage. Thus, it is preferred that the one or more linker molecules are proteolytically cleavable linker molecules. The protease for the proteolytically cleavage may be selected from the group consisting of a tobacco etch virus protease, a V8 protease, and an endonucleases for single stranded DNA.

In further preferred embodiments according to the invention, the one or more molecules capable of binding a T-cell receptor and/or the one or more molecules comprising an extracellular domain of SLAMF7 are biotinylated.

In such embodiments, the one or more linker molecules are preferably anti-biotin antibodies, the one or more molecules capable of binding a T-cell receptor are preferably biotinylated and the one or more molecules comprising an extracellular domain of SLAMF7 are preferably biotinylated.

Thus, in this embodiment the one or more molecules capable of binding a T-cell receptor and/or the one or more molecules comprising an extracellular domain of SLAMF7 are bound to the anti-biotin antibody via their biotin moiety, i.e. via non-covalent binding.

In such embodiments it is further preferred that the anti-biotin antibody is non-covalently attached to the surface of the particle via sulphate residues as activated surface moiety. This specific combination allows for the correct orientation of the SLAMF7 and T-cell receptor binding molecules at the surface of the particle and/or for the correct distance and arrangement of the SLAMF7 and T-cell receptor binding molecules towards each other and their respective target molecules on the T cell. Furthermore, this binding modality confers the ability to induce cross-linking of the target molecules.

In another preferred embodiment of the invention, the anti-biotin antibody is directly attached to the surface of the particle via tosyl residues as activated surface moiety. Another possibility of covalently binding is using carbodiimide reaction in combination with carboxylate-modified particles. Another reaction involves carboxylate-modified particles that are bound with active ester groups as an intermediate. A direct loading of particles with SLAMF7 and T-cell receptor binding molecules is also achieved by chloromethyl-activated particles made of sytrene / vinylbenzylchloride copolymers. These specific binding modalities increase the stability of the coated particles and prevents detachment of bound SLAMF7 and T-cell receptor interacting molecules.

In other preferred embodiments, the biotinylated molecules capable of binding a T-cell receptor and/or the biotinylated molecules comprising an extracellular domain of SLAMF7 are covalently attached to the linker molecules, wherein the linker molecule is avidin or a derivative thereof, preferably neutravidin, or streptavidin or derivatives thereof.

It is further preferred that the one or more molecules comprising an extracellular domain of SLAMF7 are selected from the group consisting of SLAMF7 molecules, anti-SLAMF7 antibodies, and recombinant SLAMF7 molecules.

The one or more molecules capable of binding a T-cell receptor are preferably selected from the group consisting of MHC I, MHC II, and anti-CD3.

It is preferred is that the one or more molecules capable of binding a T-cell receptor are MHC I or MHC II.

Preferably, the one or more molecules capable of binding a T-cell receptor further comprise a peptide.

It is preferred that said peptide is an antigen, preferably an antigen selected from the group consisting of a viral antigen, a bacterial antigen, a fungal antigen, and a tumour-related antigen.

In a preferred embodiment, the antigen is a tumour-related antigen, preferably an antigen associated with a cancer selected from the group consisting of melanoma, lung cancer, prostate cancer, breast cancer, cervical cancer, bladder cancer, any entity of leukaemia, pancreatic cancer and any cancer with known protein mutations or excessive expression of a certain molecule. The antigen is presented from the one or more molecules capable of binding a T-cell receptor.

In another preferred embodiment the antigen is a viral antigen, preferably from Coronaviruses, especially SARS-CoV-2 virus, Influenza virus strains, human immunodeficiency virus (HIV), hepatitis virus strains, Ebola viruses, Herpesviruses, Papillomaviruses, Hantaviruses, Rabiesviruses, Measles virus, Flaviviruses, Enteroviruses, Rhinoviruses, Zika viruses, or dengue viruses.

In another preferred embodiment said antigen has a low affinity to its receptor on antigen-recognising cells.

With the particles according to the invention, CD8+ T cells that are directed also against less potent antigens can be recruited and expanded, which is, as mentioned before, one of the key advantages of the present invention. The inventors could show that the antigen-specific crosslinking of the T-cell receptor and SLAMF7 according to the invention, the activation threshold of cytotoxic CD8+ T cells is lowered and cell division increased. Consequently, with the particles according to the present invention, an individually tailored immune therapy with less side effects is possible.

As mentioned before, in preferred embodiments of the invention, the particle is made of polyester, poly(methyl methacrylate), and silicate.

This enables the particles to firmly adsorb other hydrophobic molecules, such as proteins, nucleic acids, hormones or medical agents via van-der-Waals forces. Particles consisting of silica are inherently hydrophilic. Thus, as described above, the one or more molecules capable of binding a T-cell receptor and/or the one or more molecules comprising an extracellular domain of SLAMF7 according to the invention, are also, due to their inherent properties, passively bound to the particle, without any linker molecule or activated surface moiety, but just mediated by the hydrophobic character of the microparticles.

Furthermore, the particles according to the invention are preferably spherical. Preferably, the spherical particles have a diameter of between 0.05 - 10 µm. More preferably, the spherical particles have a diameter of between 0.05 - 7 µm. Even more preferably, the spherical particles have a diameter of between 2-7 µm. Still even more preferably, the spherical particles have a diameter of between 3-6 µm. Most preferably, the spherical particles have a diameter of 5 µm.

In other specific embodiments, the particles according to the invention are fluorescent. The particles can also be coloured. It can also be preferred, that the particles according to the invention are magnetic.

It is further preferred that a particle according to the invention is not cytotoxic and not immunogenic. It is further (alternatively or additionally) preferred that said particle is sterilizable.

In a second aspect, the invention relates to a composition comprising a plurality of particles of the first aspect.

The particles of the composition can be identical or different from each other. In case that the particles are different from each other, it is preferred that the difference is in the presence of different molecules capable of binding a T-cell receptor.

In one preferred embodiment, the different molecules capable of binding the T-cell receptor are selected from the group consisting of MHC I, MHC II, and anti-CD3 molecules. Preferably, the different molecules are MHC I and MHC II. This enables to provoke an immune response from different immune repertoires.

In another preferred embodiment, the difference is in the presence of different peptides loaded onto the molecules capable of binding a T-cell receptor.

It is preferred that said peptides are antigens.

Preferably, the antigens are selected from the group consisting of viral antigens, bacterial antigens, fungal antigens, and tumour-related antigens. It is preferred that said antigens in the composition according to the invention differ from each other, but are but are from the same group of antigens, i.e. from the group consisting of viral antigens, or from the group of bacterial antigens, or fungal antigens, or tumour-related antigens.

In a preferred embodiment, the antigens are tumour-related antigen, preferably an antigen associated with a cancer selected from the group consisting of melanoma, lung cancer, prostate cancer, breast cancer, cervical cancer, bladder cancer, any entity of leukaemia, pancreatic cancer and any cancer with known protein mutations or excessive expression of a certain molecule.

In another preferred embodiment the antigens in the composition are derived from a specific virus, or a specific bacteria or a specific fungus.

In a preferred embodiment, the antigens are viral antigens, preferably from a SARS-CoV-2 virus or other Influenza strains.

In preferred embodiments, the different antigens in the composition according to the invention are different antigens from a specific virus. Preferably, the specific virus is the SARS-CoV-2 virus. This enables to provoke a stronger immune response against SARS-CoV-2.

In other embodiments according to the invention it is preferred that the antigens are different viral antigens, from different viral strains of the same family. Preferably, the different antigens are from different Influenza strains, but all contained in the same composition. This enables to provoke a broad immune response against various types of Influenza viruses.

It can also be preferred that the antigens are different from each other but all relate to the same type of cancer. This enables to provoke an immune response against a certain tumour, approaching from different pathways.

In a preferred embodiment, the composition is a pharmaceutical composition. The pharmaceutical composition is administrated in a therapeutically effective amount.

It is preferred that a composition or a pharmaceutical composition according to the invention further comprises one or more pharmaceutically acceptable carriers, diluents, and/or excipients. An adjuvant may additionally be present.

Pharmaceutically acceptable carriers or diluents for therapeutic use are well known in the pharmaceutical art, and are described, for example, in Remington's Pharmaceutical Sciences, Mack Publishing Co. (A. R Gennaro edit. 1985). Examples of suitable carriers include, for example, magnesium carbonate, magnesium stearate, talc, sugar, lactose, pectin, dextrin, starch, gelatin, tragacanth, methylcellulose, sodium carboxymethylcellulose, a low melting wax, cocoa butter, and the like. Examples of suitable diluents include ethanol, glycerol, and water.

Pharmaceutical carriers, diluents, and/or excipients can be selected with regard to the intended route of administration and standard pharmaceutical practice. The pharmaceutical compositions of the present invention may comprise as, or in addition to, the carrier(s), excipient(s) or diluent(s) any suitable binder(s), lubricant(s), suspending agent(s), coating agent(s), and/or solubilizing agent(s). Examples of suitable binders include starch, gelatin, natural sugars such as glucose, anhydrous lactose, free-flow lactose, beta-lactose, corn sweeteners, natural and synthetic gums, such as acacia, tragacanth or sodium alginate, carboxymethyl cellulose, and polyethylene glycol. Examples of suitable lubricants include sodium oleate, sodium stearate, magnesium stearate, sodium benzoate, sodium acetate, sodium chloride, and the like. Preservatives, stabilizers, dyes, and even flavoring agents may be provided in the pharmaceutical composition. Examples of preservatives include sodium benzoate, sorbic acid, and esters of p-hydroxybenzoic acid. Antioxidants and suspending agents may be also used.

A pharmaceutical composition according to the invention will be useful for treating, preventing, or reducing the severity of a disease or disorder. It may be administered locally or systemically. It is preferred that the pharmaceutical composition is formulated for local administration or systemic administration. In particular, the local administration is by parenteral administration, e.g. by intravenous administration, subcutaneous administration, intradermal administration, intramuscularly administration, and the systemic administration is by intraarterial administration. Preferably the composition is administered subcutaneously, intradermally, or intramuscularly.

In a third aspect, the present invention relates to a particles of the first aspect or a composition of the second aspect for use as a medicament.

Said medicament can be used as only medicament, i.e. for single therapy.

In one embodiment, the particle of the first aspect or the composition of the second aspect is used as medicament when patient treated is not responding to regular treatment (e.g. as substitution therapy).

In a fourth aspect, the present invention relates to a combination comprising the particle of the first aspect or the composition of the second aspect and a drug different from the particle or composition for use as a medicament.
This means, that the particle of the first aspect or composition according to the second aspect can not only be used in single therapy as described above, but also in combination therapy with another drug. This may be helpful to combat side effects of a single-drug therapy and/or to approach the disorder or disease from different pathway. Furthermore, combination therapy allows to precisely potentiate the natural defence against a disorder such as cancer and thus, provide lifelong protection against metastases. For example, such a combination therapy can include the administration of the particle of the first aspect or the composition of the second aspect with conventional immune checkpoint inhibitors like anti-CTLA-4 or anti-PD-1 antibodies.
The particle or composition and the drug different from the particle or composition can be administered concurrently or consecutively.

In a fifth aspect, the present invention relates to a particle of the first aspect or a composition of the second aspect for use in the treatment of cancer, an infection, or an inflammation. Thus, the particle of the first aspect or the composition of the second aspect are used in a single therapy.

The cancer is preferably selected from the group consisting of melanoma, lung cancer, prostate cancer, breast cancer, cervical cancer, bladder cancer, any entity of leukaemia, pancreatic cancer and any cancer with known protein mutations or excessive expression of a certain molecule. The infection is preferably selected from the group consisting of a viral infection, preferably an infection caused by SARS-CoV-2 virus or other Influenza strains, a fungal infection, and a bacterial infection, e.g. a bacterial infection causing Sepsis.

In a sixth aspect, the present invention relates to a combination comprising the particle of the first aspect or the composition of the second aspect and a drug different from the particle or composition for use in the treatment of cancer, an infection, or an inflammation. Thus, the particle of the first aspect or the composition of the second aspect are also used in a combination therapy. The particle or composition and the drug different from the particle or composition can be administered concurrently or consecutively.

Preferably, the drug different from the particle or composition is selected from the group consisting of an immunomodulatory agent, a chemotherapeutic agent, an immunotherapeutic agent, an immunosuppressive agent, cytostatic agent, an antibody, and an antibiotic. Alternatively, the therapy with the particle or composition can be combined with radiotherapy.

The cancer is preferably selected from the group consisting of melanoma, lung cancer, prostate cancer, breast cancer, cervical cancer, bladder cancer, any entity of leukaemia, pancreatic cancer and any cancer with known protein mutations or excessive expression of a certain molecule. The infection is preferably selected from the group consisting of a viral infection, preferably an infection caused by SARS-CoV-2 virus or other Influenza strains, a fungal infection, and a bacterial infection, e.g. a bacterial infection causing Sepsis.

In a seventh aspect, the invention relates to a kit comprising the particle of the first aspect or the composition of the second aspect.

The kit may further comprise a drug different from the particle or composition. The kit may alternatively comprise a combination of the particle of the first aspect or the composition of the second aspect and a drug different from the particle or composition.

Preferably, the drug different from the particle or composition is selected from the group consisting of an immunomodulatory agent, a chemotherapeutic agent, a cytotoxic agent, an immunotherapeutic agent, an immunosuppressive agent, an antibody, and an antibiotic.

In one preferred embodiment, the kit comprises
(i) a packaging material, and
(ii) the particle of the first aspect or the composition of the second aspect, and
(iii) optionally a drug different from the particle or composition.

In one more preferred embodiment, the kit further comprises
(iv) a label or packaging insert contained within the packaging material indicating that subjects receiving the components comprised therein can be treated for cancer, an infection, or an inflammation.

In one even more preferred embodiment, the label or packaging insert further comprises the information that the dose at which the particle of the first aspect or composition of the second aspect and optionally the drug different from the particle or composition is to be administered.

In an eighth aspect, the present invention relates to a vaccine comprising the particle of the first aspect or the composition of the second aspect.

The subject receiving the vaccine may be healthy, i.e. the vaccine is for immunization or vaccination. The subject may also be diseased (i.e. a patient), e.g. suffering from cancer, and the vaccination is for curing the disease.

It is preferred that the vaccine is used for inducing an immune response or for immune therapy such as immunization or vaccination, or as an anti-cancer medicament.

In one embodiment of the invention, it is preferred that an immune response against cancer is induced. The cancer can be, for example, carcinoma, lymphoma, blastoma, sarcoma, or leukemia. More particularly, the cancer can be, for example, bone cancer, blood cancer lung cancer, liver cancer, pancreatic cancer, skin cancer, cancer of the head or neck, cutaneous or intraocular melanoma, uterine cancer, ovarian cancer, rectal cancer, cancer of the anal region, stomach cancer, colon cancer, breast cancer, prostate cancer, uterine cancer, carcinoma of the sexual and reproductive organs, Hodgkin's Disease, cancer of the esophagus, cancer of the small intestine, cancer of the endocrine system, cancer of the thyroid gland, cancer of the parathyroid gland, cancer of the adrenal gland, sarcoma of soft tissue, cancer of the bladder, cancer of the kidney, renal cell carcinoma, carcinoma of the renal pelvis, neoplasms of the central nervous system (CNS), neuroectodermal cancer, spinal axis tumors, glioma, meningioma, or pituitary adenoma.

It is further preferred that the cancer selected from the group consisting of melanoma, lung cancer, prostate cancer, breast cancer, cervical cancer, bladder cancer, any entity of leukaemia, pancreatic cancer and any cancer with known protein mutations or excessive expression of a certain molecule.

It is further (alternatively or additionally) preferred that an immune response against an infectious disease is induced.

An infectious disease can be, for example, a viral disease, a bacterial disease, a fungal disease or a parasitic disease. Preferably, the infectious disease is an infection caused by the SARS-CoV-2 virus or other Influenza strains, a fungal infection, a bacterial infection, or bacterial infection causing Sepsis. Preferably, the infection is caused by the SARS-CoV-2 virus. It can also be preferred that the infection is caused by a different Influenza virus, human immunodeficiency virus, hepatitis virus, Herpesvirus, Papillomavirus, Hantavirus, Rabiesvirus, Measles virus, Flavivirus, Enterovirus, Rhinovirus, Zika virus, dengue virus, or a different Staphylococcus, Mycobacterium, Salmonella, Neisseria, Helicobacter, or Serratia bacterial strain.

The induction of an immune response may result in the immunization or vaccination of the treated subject/patient.

In a ninth aspect, the invention relates to the use of the particle of the first aspect or the composition of the second aspect for *ex-vivo* expansion and/or activation of MHC recognising (and SLAMF7 expressing) cells. As to the preferred embodiments, it is referred to the first and/or second aspect of the present invention.

The MHC recognising (and SLAMF7 expressing) cells referred to in the ninth aspect of the present invention are preferably selected from the group consisting of CD8+ and CD4+ T-cells, or NK cells. More preferably, the T-cells are selected from the group consisting of CD8+ cytotoxic T-cells. The subject that donates the T-cells may be diseased (i.e. a patient), e.g. suffering from cancer, and the *ex-vivo* expansion is for curing the disease. It is preferred that under this aspect of the invention, the contacting is carried out *in-vitro* under conditions allowing MHC recognising (and SLAMF7 expressing) T cells to bind to the particle.

Thus, a further application aspect of the particles of the present invention, is the use for *ex-vivo* activation and enrichment of CD8+ T cells to perform an adoptive T-cell transfer. The particles of the present invention lead to an antigen-specific crosslinking of the T-cell receptor and SLAMF7, which lowers the activation threshold and increases the cell division of CD8+ T cells. This effect of the particles enables the recruitment and proliferation of CD8+ T cells that are directed against less potent antigens and thus exploit the full range of the immune response. Thus, these expanded low-affinity T cells that otherwise could not be induced in the donating patient can then be re-applicated the treat cancer or infections.

In a tenth aspect, the invention relates to a method of expanding the population of MHC recognising (and SLAMF7 expressing) cells comprising the steps of contacting a particle of the first aspect of the invention and a sample suspected of comprising MHC recognising (and SLAMF7 expressing) cells, and isolating the MHC recognising (and SLAMF7 expressing) cells bound to the particle.

It is preferred that under this aspect of the invention, the contacting is carried out under conditions allowing MHC recognising (and SLAMF7 expressing) cells to bind to the particle.

Thus, a further application aspect of the particles of the present invention, is the use for *ex-vivo* activation and enrichment of CD8+ T cells. The particles of the present invention lead to an antigen-specific crosslinking of the T-cell receptor and SLAMF7, which lowers the activation threshold and increases the cell division of CD8+ T cells. This effect of the particles enables the recruitment and proliferation of CD8+ T cells that are directed against less potent antigens and otherwise would stay inactivated and not detectable. As a result of specific binding of the T cells to the particles these interactions can be used to enrich and isolate the MHC recognising (and SLAMF7 expressing) cells. e.g. by magnetically sorting of the (expanded) T cells or the attached particles, or both.

Additionally, the particles of the invention can be used as tool in research to visualize even very minor and inconspicuous T-cell responses, that were otherwise not measurable.

Thus, in an eleventh aspect, the invention relates to a method for detecting or monitoring the presence and/or activation of MHC recognising (and SLAMF7 expressing) cells comprising the steps of contacting a particle of the first aspect of the invention and a sample (suspected of) comprising MHC recognising (and SLAMF7 expressing) cells, and determining any binding of the particle to the MHC recognising (and SLAMF7 expressing) cells, whereby the binding indicates the presence/activation of MHC recognising (and SLAMF7 expressing) cells.

In a twelfth aspect, the invention is related to a method for establishing a prognosis of a disease, determining the status of a disease, or diagnosing a disease involving MHC recognising (and SLAMF7 expressing) cells comprising the steps of contacting a particle of the first aspect of the invention and a sample (suspected of) comprising MHC recognising (and SLAMF7 expressing) cells, and determining any binding of the particle to the MHC recognising (and SLAMF7 expressing) cells, thereby establishing a prognosis of a disease, determining the status of a disease, or diagnosing a disease involving MHC recognising (and SLAMF7 expressing) cells.

It is preferred that said disease is selected from the group consisting of cancer, an infection, or an inflammation. The cancer can be, for example, carcinoma, lymphoma, blastoma, sarcoma, or leukemia. More particularly, the cancer can be, for example, bone cancer, blood cancer lung cancer, liver cancer, pancreatic cancer, skin cancer, cancer of the head or neck, cutaneous or intraocular melanoma, uterine cancer, ovarian cancer, rectal cancer, cancer of the anal region, stomach cancer, colon cancer, breast cancer, prostate cancer, uterine cancer, carcinoma of the sexual and reproductive organs, Hodgkin's Disease, cancer of the esophagus, cancer of the small intestine, cancer of the endocrine system, cancer of the thyroid gland, cancer of the parathyroid gland, cancer of the adrenal gland, sarcoma of soft tissue, cancer of the bladder, cancer of the kidney, renal cell carcinoma, carcinoma of the renal pelvis, neoplasms of the central nervous system (CNS), neuroectodermal cancer, spinal axis tumors, glioma, meningioma, or pituitary adenoma.

It is preferred that the cancer is selected from the group consisting of melanoma, lung cancer, prostate cancer, breast cancer, cervical cancer, bladder cancer, any entity of leukaemia, pancreatic cancer and any cancer with known protein mutations or excessive expression of a certain molecule.

If the disease is an infection, it is preferred that the infection is selected from the group consisting of a viral infection, preferably an infection caused by SARS-CoV-2 virus or other Coronavirus strains, Influenza strains, a fungal infection, and a bacterial infection, or bacterial infection causing Sepsis. Preferably, the disease is caused by an Influenza strain or different Influenza strains. More preferably, the infection is caused by SARS-CoV-2. It can also be preferred that the infection is caused by human immunodeficiency virus, hepatitis virus, Herpesvirus, Papillomavirus, Hantavirus, Rabiesvirus, Measles virus, Flavivirus, Enterovirus, Rhinovirus, Zika virus, dengue virus, or a different Staphylococcus, Mycobacterium, Salmonella, Neisseria, Helicobacter, or Serratia bacterial strain.

In a thirteenth aspect, the invention relates to a method for determining the effectiveness of a medicament against a disease involving MHC recognising (and SLAMF7 expressing) cells comprising the steps of contacting a particle of the first aspect of the invention and a sample (suspected of) comprising MHC recognising (and SLAMF7 expressing), determining any binding of the particle to the MHC recognising (and SLAMF7 expressing) cells, thereby determining the effectiveness of the medicament.

It is preferred that said disease is selected from the group consisting of cancer, an infection, or an inflammation. The cancer can be, for example, carcinoma, lymphoma, blastoma, sarcoma, or leukemia. More particularly, the cancer can be, for example, bone cancer, blood cancer lung cancer, liver cancer, pancreatic cancer, skin cancer, cancer of the head or neck, cutaneous or intraocular melanoma, uterine cancer, ovarian cancer, rectal cancer, cancer of the anal region, stomach cancer, colon cancer, breast cancer, prostate cancer, uterine cancer, carcinoma of the sexual and reproductive organs, Hodgkin's Disease, cancer of the esophagus, cancer of the small intestine, cancer of the endocrine system, cancer of the thyroid gland, cancer of the parathyroid gland, cancer of the adrenal gland, sarcoma of soft tissue, cancer of the bladder, cancer of the kidney, renal cell carcinoma, carcinoma of the renal pelvis, neoplasms of the central nervous system (CNS), neuroectodermal cancer, spinal axis tumors, glioma, meningioma, or pituitary adenoma.

It is preferred that the cancer is selected from the group consisting of melanoma, lung cancer, prostate cancer, breast cancer, cervical cancer, bladder cancer, any entity of leukaemia, pancreatic cancer and any cancer with known protein mutations or excessive expression of a certain molecule.

If the disease is an infection, it is preferred that the infection is selected from the group consisting of a viral infection, preferably an infection caused by SARS-CoV-2 virus or other Influenza strains, a fungal infection, and a bacterial infection, or bacterial infection causing Sepsis. Preferably, the disease is caused by an Influenza strain or different Influenza strains. More preferably, the infection is caused by SARS-CoV-2.

It is preferred in any of the methods described above, the sample is a biological sample obtained from a subject or a cell culture sample.

In certain embodiments of the invention, the method is carried out *ex-vivo* or *in-vitro.*

The MHC recognising (and SLAMF7 expressing) cells referred to in the tenth to thirteenth aspect of the present invention are preferably selected from the group consisting of CD8+ and CD4+ T-cells, or NK-cells. More preferably, the T-cells are selected from the group consisting of CD8+ cytotoxic T-cells.

In the following, the present invention is summarized as follows:
1. A particle comprising one or more molecules capable of binding a T-cell receptor and one or more molecules comprising an extracellular domain of SLAMF7.
2. The particle of item 1, wherein the one or more molecules capable of binding a T-cell receptor and/or the one or more molecules comprising an extracellular domain of SLAMF7 are attached to the surface of the particle.
3. The particle of item 2, wherein the one or more molecules capable of binding a T-cell receptor and/or the one or more molecules comprising an extracellular domain of SLAMF7 are directly or indirectly attached to the surface of the particle.
4. The particle of item 3, wherein the one or more molecules capable of binding a T-cell receptor and/or the one or more molecules comprising an extracellular domain of SLAMF7 had been directly attached to the surface of the particle by a reaction of activated surface moieties with said one or more molecules capable of binding a T-cell receptor and/or the one or more molecules comprising an extracellular domain of SLAMF7.
5. The particle of item 4, wherein the direct attachment to the surface of the particle via activated surface moieties is a covalent or non-covalent linkage.
6. The particle of items 4 or 5, wherein the activated surface moieties are selected from the group consisting of sulfate-, tosyl-, carboxyl-, amino-, hydroxyl-, hydrazide-, chloromethyl-, silanol-, and maleimide-moieties.
7. The particle of any one of items 3 to 6, wherein the one or more molecules capable of binding a T-cell receptor and/or the one or more molecules comprising an extracellular domain of SLAMF7 are indirectly attached to the surface of the particle via one or more linker molecules.
8. The particle of item 7, wherein the one or more linker molecules had been directly attached to the surface of the particle by a reaction of activated surface moieties with said one or more linker molecules.
9. The particle of item 8, wherein the direct attachment to the surface of the particle via activated surface moieties is a covalent or non-covalent linkage.
10. The particle of items 8 or 9, wherein the activated surface moieties are selected from the group consisting of sulfate-, tosyl-, carboxyl-, amino-, hydroxyl-, hydrazide-, chloromethyl-, silanol-, and maleimide-moieties.
11. The particle of any one of items 8 to 10, wherein the one or more linker molecules are directly attached to the one or more molecules capable of binding a T-cell receptor and/or the one or more molecules comprising an extracellular domain of SLAMF7.
12. The particle of item 11, wherein the direct attachment to the one or more molecules capable of binding a T-cell receptor and/or the one or more molecules comprising an extracellular domain of SLAMF7 is a covalent or non-covalent linkage.
13. The particle of any one of items 7 to 12, wherein the one or more linker molecules are selected from the group consisting of antibodies, antibody fragments or derivatives thereof, photoreactive conjugates, cleavable linkers, recombinant proteins, and recombinant fusion proteins.
14. The particle of item 13, wherein
   the antibodies are monoclonal antibodies, preferably anti-biotin antibodies, or polyclonal antibodies,
   the recombinant proteins are avidin and derivatives thereof, preferably neutravidin, or streptavidin and derivatives thereof, or
   the recombinant fusion protein is protein A/G.
15. The particle of any one of items 7 to 14, wherein the one or more linker molecules are proteolytically cleavable.
16. The particle of any one of items 1 to 15, wherein the one or more molecules capable of binding a T-cell receptor and/or the one or more molecules comprising an extracellular domain of SLAMF7 are biotinylated.
17. The particle of item 16, wherein the one or more linker molecules are anti-biotin antibodies, the one or more molecules capable of binding a T-cell receptor are biotinylated and the one or more molecules comprising an extracellular domain of SLAMF7 are biotinylated.
18. The particle of any one of items 1 to 17, wherein the one or more molecules comprising an extracellular domain of SLAMF7 are selected from the group consisting of SLAMF7 molecules, anti-SLAMF7 antibodies, and recombinant SLAMF7 molecules.
19. The particle of any one of items 1 to 18, wherein the one or more molecules capable of binding a T-cell receptor are selected from the group consisting of MHC I, MHC II, and anti-CD3 molecules.
20. The particle of any one of items 1 to 19, wherein the one or more molecules capable of binding a T-cell receptor further comprise a peptide.
21. The particle of item 20, wherein said peptide is an antigen, preferably, preferably an antigen selected from the group consisting of a viral antigen, a bacterial antigen, a fungal antigen, and a tumour-related antigen.
22. The particle of any one of items 1 to 21, wherein said particle is composed of/made of/comprises polyester, poly(methyl methacrylate), and silicate.
23. The particle of any one of items 1 to 22, wherein said particle is spherical.
24. The particle of item 23, wherein said spherical particle has a diameter of between 0.5 - 10 µm, preferably of between 2 to 7 µm, more preferably of between 3-6 µm, and most preferably of 5 µm.
25. A composition comprising a plurality of particles of any one of items 1 to 24.
26. The composition of item 25, wherein the composition is a pharmaceutical composition.
27. The composition of items 25 or 26, wherein the composition further comprises one or more pharmaceutically acceptable carriers, diluents, and/or excipients.
28. A particle of any one of items 1 to 24 or a composition of any one of items 25 to 27 for use as a medicament.
29. A combination comprising the particle of any one of items 1 to 24 or the composition of any one of items 25 to 27 and a drug different from the particle or composition for use as a medicament.
30. A particle of any one of items 1 to 24 or a composition of any one of items 25 to 27 for use in the treatment of cancer, an infection, or an inflammation.
31. A combination comprising the particle of any one of items 1 to 24 or the composition of any one of items 25 to 27 and a drug different from the particle or composition for use in the treatment of cancer, an infection, or an inflammation.
32. The particle or composition of item 30 or the combination of item 31, wherein
   the cancer is selected from the group consisting of melanoma, lung cancer, prostate cancer, breast cancer, cervical cancer, bladder cancer, any entity of leukaemia, pancreatic cancer and any cancer with known protein mutations or excessive expression of a certain molecule, the infection is selected from the group consisting of a viral infection, preferably an infection caused by Sars-CoV-2 virus or other Influenza strains, a fungal infection, and a bacterial infection, or bacterial infection causing Sepsis.
33. A kit comprising the particle of any one of items 1 to 24 or the composition of any one of items 25 to 27.
34. A vaccine comprising the particle of any one of items 1 to 24 or the composition of any one of items 25 to 27.
35. Use of the particle of any one of items 1 to 24 or the composition of any one of items 25 to 27 for *ex vivo* expansion and/or activation of MHC recognising (and SLAM7 expressing) cells.
36. A method of expanding the population of MHC recognising (and SLAM7 expressing) cells comprising the steps of:
   (a) contacting a particle of any one of items 1 to 24 and a sample (suspected of) comprising MHC recognising (and SLAM7 expressing) cells, and
   (b) isolating the MHC recognising (and SLAM7 expressing) cells bound to the particle.
37. The method of item 36, wherein the contacting is carried out under conditions allowing MHC recognising (and SLAM7 expressing) cells to bind to the particle.
38. A method for detecting or monitoring the presence and/or activation of MHC recognising (and SLAMF7 expressing) cells comprising the steps of:
   (a) contacting a particle of any one of items 1 to 24 and a sample (suspected of) comprising MHC recognising (and SLAMF7 expressing) cells, and
   (b) determining any binding of the particle to the MHC recognising (and SLAMF7 expressing) cells, whereby the binding indicates the presence/activation of MHC recognising (and SLAMF7 expressing) cells.
39. A method for establishing a prognosis of a disease, determining the status of a disease, or diagnosing a disease involving MHC recognising (and SLAMF7 expressing) cells comprising the steps of:
   (a) contacting a particle of any one of items 1 to 24 and a sample (suspected of) comprising MHC recognising (and SLAMF7 expressing) cells, and
   (b) determining any binding of the particle to the MHC recognising (and SLAMF7 expressing) cells, thereby establishing a prognosis of a disease, determining the status of a disease, or diagnosing a disease involving MHC recognising (and SLAMF7 expressing) cells.
40. A method for determining the effectiveness of a medicament against a disease involving MHC recognising (and SLAMF7 expressing) cells comprising the steps of:
   (a) contacting a particle of any one of items 1 to 24 and a sample (suspected of) comprising MHC recognising (and SLAMF7 expressing),
   (b) determining any binding of the particle to the MHC recognising (and SLAMF7 expressing) cells, thereby determining the effectiveness of the medicament.
41. The method of any one of items 36 to 40, wherein the sample is a biological sample obtained from a subject or
   a cell culture sample.
42. The method of any one of items 36 to 41, wherein the method is carried out *ex vivo* or *in-vitro.*

Various modifications and variations of the invention will be apparent to those skilled in the art without departing from the scope of invention. Although the invention has been described in connection with specific preferred embodiments, it should be understood that the invention as claimed should not be unduly limited to such specific embodiments. Indeed, various modifications of the described modes for carrying out the invention which are obvious to those skilled in the art in the relevant fields are intended to be covered by the present invention.

The following figures and examples are merely illustrative of the present invention and should not be construed to limit the scope of the invention as indicated by the appended claims in any way.

### BRIEF DESCRIPTION OF THE FIGURES

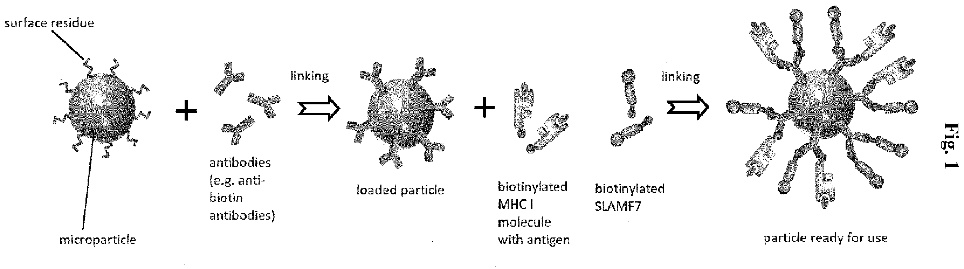

### FIGURE 1: Production of preferred particles according to the invention

It shows schematically the steps for the preparation of particles of a preferred embodiment according to the invention. Solid particles with activated surface residues are coupled with antibodies (e.g. anti-biotin antibodies). Subsequently, biotinylated MHC molecules (e.g. MHC I), loaded with antigens, and biotinylated SLAMF7 molecules, are coupled to the anti-biotin antibodies on the microparticles. The coupling of SLAMF7 and MHC molecules via antibodies to the microparticles, allows for the specific orientation of the MHC and SLAMF7 molecules.

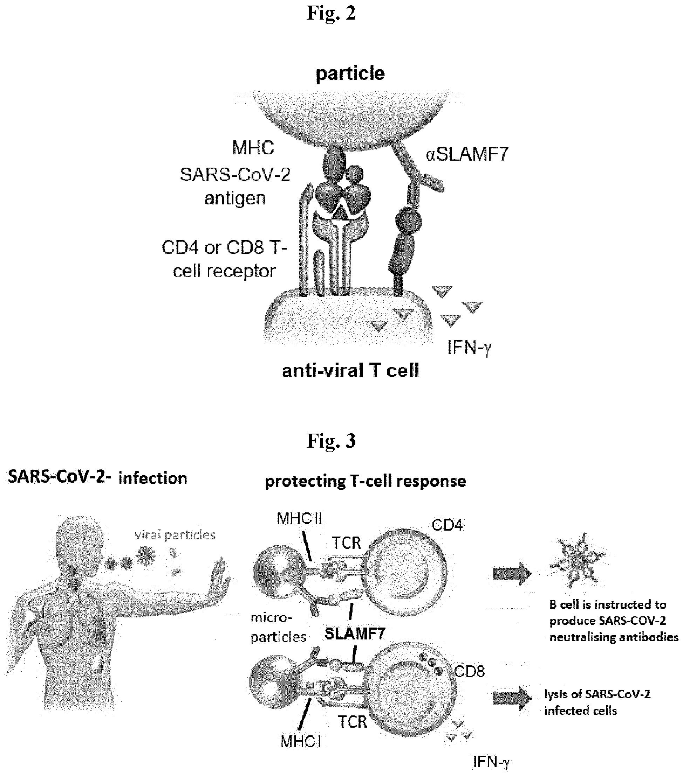

### FIGURE 2: Schematic view of the ex vivo activation of anti-viral T-cells

It shows exemplified a graphic of the activation of anti-viral T-cells via particles according to the invention. The particles are coupled to peptide-loaded MHC molecules (e.g. a control peptide or a SARS-CoV-2-antigen) and molecules comprising an extracellular domain of SLAMF7 (e.g. anti-SLAMF7 antibodies).

### FIGURE 3: Particles according to the invention can be used in the treatment of infectious diseases such as COVID-19

Infections with viruses from the *Coronaviridae* family can lead to severe and even fatal respiratory diseases. The pathology COVID-19 is caused by the novel coronavirus SARS-CoV-2. A fast and effective adaptive immune response is a prerequisite for the cure of a viral infection. As central players, T cells destroy virus-infected host cells and regulate the production of virus-neutralizing antibodies to prevent the proliferation of viruses. The underlying mechanisms and interactions in the defense against corona viruses by T cells ultimately determine the outcome of such infections, but these are poorly understood. With the help of many different particles according to the invention (different MHC haplotypes with bound SARS-CoV-2 peptides plus molecules comprising an extracellular domain of SLAMF7 as co-stimulatory molecules) specific SARS-CoV-2 T-cell responses can be visualized and used for detailed analysis, diagnostics or new therapeutic approaches. The approach will also be applicable for cancer studies and therapies.

### FIGURE 4: SLAMF7-activated CD8+ T-cells show a highly elevated cell proliferation

It shows the dilution of a fluorescent dye, that was applied to the T cells prior activation, as a result of the increase in T-cell proliferation after stimulation with particles according to the invention.

### FIGURE 5: Activated cytotoxic CD8+ T-cells show an increased expression of SLAMF7

Naive CD8+ T cells were activated with microparticles or left unactivated (control) and SLAMF7 surface expression was assessed. Whereas control cells remained negative for SLAMF7 expression, activated T cells express SLAMF7 on their surface.

### FIGURE 6: SLAMF7 activation leads to an increase in the production of the effector molecule IFN-γ

Naive CD8+ T cells were left inactivated (control) or were activated with the invention (MHC I with bound peptides plus molecules comprising an extracellular domain of SLAMF7 or not (activated)) and the IFN-γ production of these cells was assessed. T cells that received a SLAMF7 signal by the invention expressed significantly higher amounts of IFN-γ (4 biological replicates).

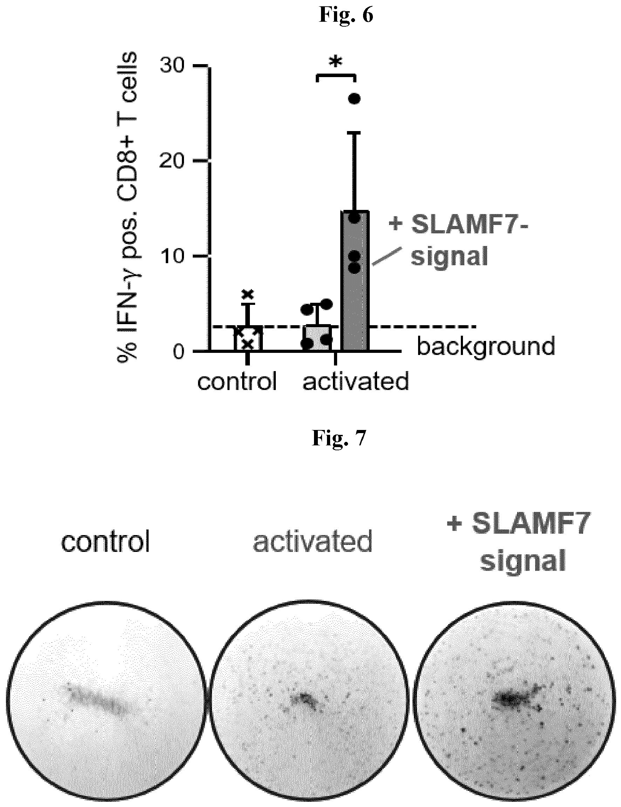

### FIGURE 7: SLAMF7 signaling leads to an increased aggregation of murine CD8+ T cells

SLAMF7 engagement leads to enhanced secondary interactions during CD8+ T-cell activation: Figure 7 shows naive CD8+ T cells that were left inactivated (control) or were activated with the invention (particles with T-cell activating molecules plus molecules comprising an extracellular domain of SLAMF7 or not (activated)). T cells that received a SLAMF7 signal formed increased numbers of clusters and bigger clusters.

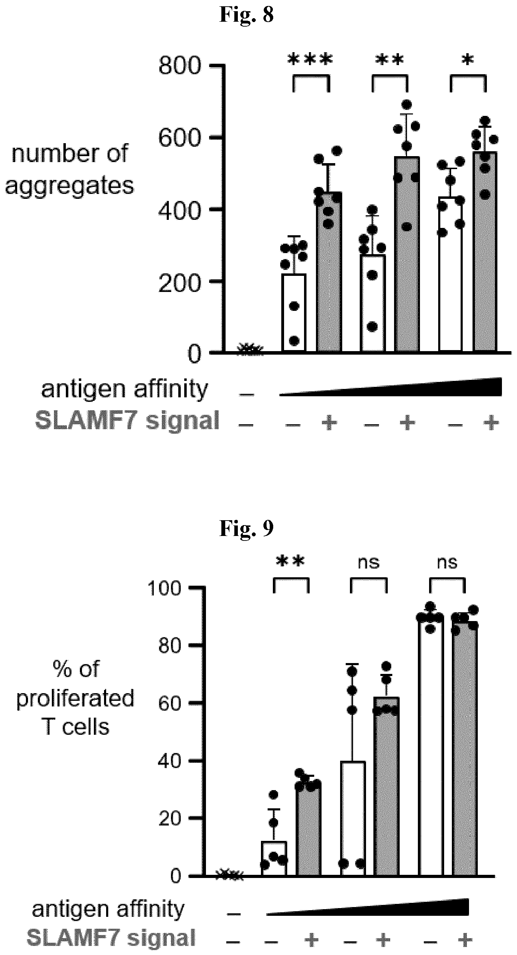

### FIGURE 8: SLAMF7 increases the aggregation behavior of murine CD8+ T-cells in response to different antigen affinities

Naive CD8+ T cells were left inactivated (x, left) or were activated with the invention (MHC I with bound peptides of different affinities plus molecules comprising an extracellular domain of SLAMF7 (grey bars) or not (open bars)) and the numbers of aggregates of these T cells were assessed. T cells that received a SLAMF7 signal by the invention formed significantly more aggregates (7 biological replicates).

### FIGURE 9: SLAMF7 increases the proliferation of murine CD8+ T-cells in response to low-affinity antigens

Naive CD8+ T cells were left inactivated (x, left) or were activated with the invention (MHC I with bound peptides of different affinities plus molecules comprising an extracellular domain of SLAMF7 (grey bars) or not (open bars)) and the percentages of the proliferated T cells were assessed. T cells that received a SLAMF7 signal by the invention show significantly elevated proliferation to low affinity antigens (5 biological replicates).

### EXAMPLES

The examples given below are for illustrative purposes only and do not limit the invention described above in any way.

### Example 1: Coating of particles coated with activated surface moieties and/or linker molecules

**Figure 1** shows schematically the steps for the preparation of particles of a preferred embodiment according to the invention. More specifically, to obtain these preferred particles according to the invention, microparticles with activated surface residues are loaded in a first step with anti-biotin antibodies and in a second step with one or more biotinylated molecules capable of binding a T-cell receptor and/or the one or more biotinylated molecules comprising an extracellular domain of SLAMF7.

### Example 2: Particles according to the invention critically influence the differentiation commitment of cytotoxic T cells

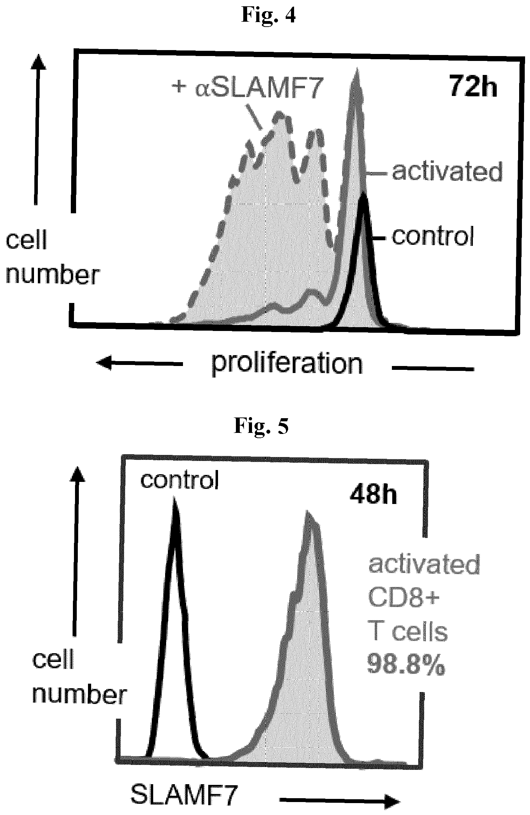

SLAM F7 crosslinking on activated CD8+ T cells promotes contact formation (**Figures 7** and **8**) and proliferation (**Figures 4** and **9**), particularly in response to weak antigenic stimulation. Furthermore, SLAMF7 is strongly induced in cytotoxic T cells (**Figure 5**).

### Mice

OVA-specific TCRtg mice (OT-I) and C57BL/6 mice were bred under specific pathogen-free conditions in the central animal facility of the University of Magdeburg Medical School (Germany). OT-I TCRtg surface expression was controlled by flow cytometry. All animal experiments were performed in accordance with institutional, state and federal guidelines.

### Antibodies and primer

The following antibodies against were used: *α* SLAMF7 (4G2), *α* SLAMF7 isotype control antibody (RTK2071), *α* Vα2-TCR (B20.1), *α* CD3 (145-2C11), *α* CD28 (37.51), *α* CD8a (53-6.7).

### Primer: SLAMF7 real-time, SLAMF7-long Isoform

### Cell isolation and stimulation

Isolation of naive CD8⁺ CD44^{low} CD62L^{high} T cells from spleens, inguinal- and axillary-lymph nodes was performed with *α* CD8a-FITC and *α* FITC microbeads (Miltenyi Biotec, Bergisch Gladbach, Germany) according to the manufacturer's instructions. Isolated CD8⁺ T cells were stimulated with antibodies or antibodies in combination with 0.5 µg/ml recombinant CD80-Fc (BioLegend) and 0.1 µg/ml antigen pulsed H-2Kb:Ig (DimerX I, BD Biosciences) molecules immobilized on microspheres (Thermo Fisher Scientific, Waltham, MA, USA). Microspheres were coated as described elsewhere (Krummel 1995). Antigens were used as high affinity SIINFEKL (N4, InvivoGen, Toulouse, France), low affinity SIITFEKL (T4, AnaSpec, Fremont, USA), and very low affinity SIIVFEKL (V4, DGpeptides, Hangzhou, China). Cells were cultured with complete medium (RPMI, with 100 U/ml Penicillin, 100 µg/ml Streptomycin (Thermo Fisher Scientific), and 10% FCS (Biochrom, Berlin, Germany)) supplemented with 10 ng/ml of recombinant IL-12 (BioLegend). In some experiments, CD8⁺ T cells were labelled with 2,5 µM CFSE.

### Flow cytometry

Surface staining of T cell molecules was conducted on washed T cells incubated with the respective staining antibodies.

For intracellular cytokine staining cells were treated with 5 µg/ml Brefeldin A for 4 h, then fixed and permeabilized with 0.5% saponine (Sigma-Aldrich) and incubated with the respective staining antibodies. Cytometric measurements were performed on a FACS-Canto II (BD Biosciences) and analyzed with FlowJo (Tree Star, Ashland, OR, USA) software.

### Cell Cluster Determination

Fluorescently labelled CD8+ T cells were activated in 96 well plates and imaged and counted with a ImmunoSpot analyzer (CTL, Cleveland, OH, USA).

### Statistical analysis

P-values < 0.05 were considered statistical significant and calculated with a two-sided Mann-Whitney rank sum test for the comparison of two groups; for more than two groups a Kruskal-Wallis test with Dunn's correction was used. All determined significances remained by using an equivalent parametric test. Statistical significance is indicated as followed: ns, not significant, **P*<0.05, ***P*<0.01, and ****P*<0.001.

## Claims

1. A particle comprising one or more molecules capable of binding a T-cell receptor and one or more molecules comprising an extracellular domain of SLAMF7.

2. The particle of claim 1, wherein the one or more molecules capable of binding a T-cell receptor and/or the one or more molecules comprising an extracellular domain of SLAMF7 are attached to the surface of the particle.

3. The particle of claims 1 or 2, wherein
the one or more molecules capable of binding a T-cell receptor are selected from the group consisting of MHC I, MHC II, and anti-CD3 molecules, and/or
the one or more molecules comprising an extracellular domain of SLAMF7 are selected from the group consisting of SLAMF7 molecules, anti-SLAMF7 antibodies, and recombinant SLAMF7 molecules.

4. A composition comprising a plurality of particles of any one of claims 1 to 3.

5. A particle of any one of claims 1 to 3 or a composition of claim 4 for use as a medicament.

6. A combination comprising the particle of any one of claims 1 to 3 or the composition of claim 4 and a drug different from the particle or composition for use as a medicament.

7. A particle of any one of claims 1 to 3 or a composition of any one of claim 4 for use in the treatment of cancer, an infection, or an inflammation.

8. A combination comprising the particle of any one of claims 1 to 3 or the composition of claim 4 and a drug different from the particle or composition for use in the treatment of cancer, an infection, or an inflammation.

9. A kit comprising the particle of any one of claims 1 to 3 or the composition of claim 4.

10. A vaccine comprising the particle of any one of claims 1 to 3 or the composition of claim 4.

11. Use of the particle of any one of claims 1 to 3 or the composition of claim 4 for *ex vivo* expansion and/or activation of MHC recognising (and SLAM7 expressing) cells.

12. A method of expanding the population of MHC recognising (and SLAM7 expressing) cells comprising the steps of:
(a) contacting a particle of any one of claims 1 to 3 and a sample (suspected of) comprising MHC recognising (and SLAM7 expressing) cells, and
(b) isolating the MHC recognising (and SLAM7 expressing) cells bound to the particle, wherein, preferably, the contacting is carried out under conditions allowing MHC recognising (and SLAM7 expressing) cells to bind to the particle.

13. A method for detecting or monitoring the presence and/or activation of MHC recognising (and SLAMF7 expressing) cells comprising the steps of:
(a) contacting a particle of any one of claims 1 to 3 and a sample (suspected of) comprising MHC recognising (and SLAMF7 expressing) cells, and
(b) determining any binding of the particle to the MHC recognising (and SLAMF7 expressing) cells, whereby the binding indicates the presence/activation of MHC recognising (and SLAMF7 expressing) cells.

14. A method for establishing a prognosis of a disease, determining the status of a disease, or diagnosing a disease involving MHC recognising (and SLAMF7 expressing) cells comprising the steps of:
(a) contacting a particle of any one of claims 1 to 3 and a sample (suspected of) comprising MHC recognising (and SLAMF7 expressing) cells, and
(b) determining any binding of the particle to the MHC recognising (and SLAMF7 expressing) cells, thereby establishing a prognosis of a disease, determining the status of a disease, or diagnosing a disease involving MHC recognising (and SLAMF7 expressing) cells.

15. A method for determining the effectiveness of a medicament against a disease involving MHC recognising (and SLAMF7 expressing) cells comprising the steps of:
(a) contacting a particle of any one of claims 1 to 3 and a sample (suspected of) comprising MHC recognising (and SLAMF7 expressing),
(b) determining any binding of the particle to the MHC recognising (and SLAMF7 expressing) cells, thereby determining the effectiveness of the medicament.
